# FASCICULE DE BREVET EUROPEEN

(11) **EP 3 628 654 B1**
(45) Date de publication et mention de la délivrance du brevet: **16.02.2022**
(21) Numéro de dépôt: 19199698.2
(22) Date de dépôt: 25.09.2019
(51) Int. Cl.: C07C 37/00, C07C 37/68, C07C 43/20, C10L 5/44, C10L 9/08

(54) **PROCÉDÉ ET INSTALLATION DE PRODUCTION D'EUGÉNOL PAR TORREFACTION DE BIOMASSE SUIVIE D'UNE CONDENSATION ÉTAGÉE**
VERFAHREN UND ANLAGE ZUR EUGENOL-HERSTELLUNG DURCH RÖSTEN EINER BIOMASSE UND ANSCHLIESSENDER MEHRSTUFIGER KONDENSATION
METHOD AND FACILITY FOR PRODUCING EUGENOL BY ROASTING BIOMASS FOLLOWED BY STAGED CONDENSATION

(30) Priorité: 26.09.2018 FR 1858782
(43) Date de publication de la demande: 01.04.2020
(73) Titulaire: Commissariat à l'Energie Atomique et aux Energies Alternatives, 75015 Paris (FR); Institut National Polytechnique de Toulouse, 31029 Toulouse (FR); Centre National de la Recherche Scientifique, 75016 Paris (FR); Centre de Coopération Internationale en Recherche Agronomique pour le Développement, 75116 Paris 16ème (FR)
(72) Inventeur: BOISSONNET, Guillaume, 38054 Grenoble Cedex 9 (FR); DUPONT, Capucine, 2613 EP Delft (NL); LE THANH, Kim, 30300 Beaucaire (FR); COMMANDRE, Jean-Michel, 34790 Grabels (FR); MEYER, Xuan, 31750 Escalquens (FR); MEYER, Michel, 31750 Escalquens (FR); CONDORET, Jean-Stéphane, 31400 Toulouse (FR); DESTRAC, Philippe, 31500 Toulouse (FR); GOURDON, Christophe, 31500 Toulouse (FR); DETCHEBERRY, Mylène, 44980 Sainte Luce sur Loire (FR)
(74) Mandataire: Nony

(56) Documents cités:
- US-A1- 2010 223 839
- US-A1- 2011 245 489
- US-A1- 2011 278 149
- US-A1- 2017 321 140
- LEENA FAGERNÄS ET AL: "Composition, Utilization and Economic Assessment of Torrefaction Condensates", ENERGY & FUELS., vol. 29, no. 5, 20 avril 2015 (2015-04-20) , pages 3134-3142, XP055605780, WASHINGTON, DC, US. ISSN: 0887-0624, DOI: 10.1021/acs.energyfuels.5b00004

## Description

### Domaine technique

La présente invention concerne un procédé et installation de production d'eugénol et le cas échéant de formol, solution aqueuse de formaldéhyde polymérisé.

L'invention vise en particulier à améliorer la production de ces produits qui sont à l'état gazeux, lorsqu'issus du séchage et de torréfaction de biomasse, quel que soit son type.

Par « biomasse», on entend dans le cadre de l'invention, tout matériau inhomogène d'origine végétale ou animale contenant du carbone, telle que de la biomasse ligno-cellulosique, des résidus forestiers ou agricoles (paille), qui peut être quasi-sec ou imbibé d'eau comme les déchets ménagers.

En particulier, la biomasse peut être de type ligno-cellulosique, telle que le bois et les matières agricoles et présenter n'importe quel taux d'humidité, de préférence comprenant entre 10 et 60% d'eau, et être traité par un procédé selon l'invention sous toutes formes variées tels que granulés, plaquettes...

### Etat de la technique

L'eugénol fait partie des molécules que l'on peut trouver dans certaines épices, telles que le clou de girofle.

La production traditionnelle consiste à réaliser une huile essentielle de clous de girofle, obtenue par hydrodistillation (entraînement à la vapeur d'eau) du clou du girofle ou d'autres produits naturels (noix de muscade, basilic, laurier...) de laquelle on extrait l'eugénol.

L'autre voie de production existante consiste à réaliser une synthèse de gaïacol qui lui est obtenu par voie pétrochimique.

La valorisation de la biomasse fait l'objet de nombreux travaux de recherche et de développement industriel afin d'accroitre la diversification des ressources d'énergies et de produits chimiques. Les filières de conversion thermique par gazéification et combustion sont particulièrement envisagées avec une valorisation la plus complète possible des flux de produits et d'énergie à toutes les étapes.

Les usines dites « Biomass to Liquid », désignées sous l'acronyme anglais BtL sont des usines destinées à mettre en œuvre un procédé de conversion thermochimique de la biomasse par gazéification en vue de produire un carburant liquide. Ces usines BtL ont déjà été largement décrites avec un grand nombre de données économiques tant en investissement qu'en coût de production [1]. Les usines BtL, plus généralement les usines destinées à produire un carburant gazeux ou liquide à partir de la biomasse sont actuellement des projets de démonstration qui visent à tester et valider toute la chaine technologique du procédé de conversion thermochimique. La première étape de traitement consiste en un séchage de la biomasse humide. En effet, l'humidité de la biomasse fraîchement collectée est typiquement comprise entre 30 et 50% massique, ce taux initial d'humidité variant en fonction du type de biomasse, de la période de collecte et de l'endroit où est stockée la biomasse. Il a déjà été prouvé que l'eau contenue dans la biomasse réduit fortement le rendement énergétique de gazéification du fait d'une nécessaire évaporation de cette humidité. En outre, il est d'ores et déjà admis que le séchage naturel de la biomasse ne peut être réellement envisagé dans une application industrielle compte tenu du temps nécessaire requis dans ces conditions opératoires : [2],[3].

En d'autres termes, à l'échelle industrielle, seul un séchage forcé de la biomasse peut être envisagé. Il semble de même admis que le séchage de la biomasse est une étape qui peut être énergivore et selon certaines estimations cela peut représenter jusqu'à 15% de l'énergie industrielle utilisée. En outre, l'évaporation des derniers pourcentages d'humidité augmente considérablement la consommation énergétique.

Ainsi, un séchage de la biomasse aux alentours d'un taux d'humidité de 15% avant sa gazéification est un bon compromis en termes d'efficacité de rendement de conversion et de consommation énergétique requise. La torréfaction de la biomasse de préférence ligno-cellulosique, est une étape de prétraitement de la biomasse en vue de son injection sous forme pulvérisée dans un réacteur à flux entraîné (réacteur de gazéification) ou dans un réacteur dit de co-combustion biomasse et charbon dans une centrale thermique à charbon ou en vue de sa mise sous forme de granulés. En effet, la structure fibreuse et élastique de la biomasse rend sa micronisation énergivore et confère au produit broyé des caractéristiques inadaptées à une injection sous forme pulvérisée. En matière d'application, la torréfaction est aujourd'hui également envisagée pour produire un combustible renouvelable ayant des propriétés stables dans le temps ou en fonction de la ressource et pouvant être mélangé à tout autre type de combustible solide. La torréfaction est un traitement thermique doux de la biomasse à l'interface entre le séchage et la pyrolyse, généralement réalisé à des températures comprises entre 200 et 300°C, sous atmosphère en défaut d'oxygène, et qui vise à éliminer l'eau et à modifier une partie de la matière organique de la biomasse pour casser ses fibres. Autrement dit, ce traitement thermique doux altère la structure fibreuse de la biomasse, facilitant ainsi par la suite son broyage, son transport et son injection dans un réacteur de gazéification ou de co-combustion.

Le prétraitement par torréfaction améliore également les propriétés de la biomasse en vue de son stockage en lui conférant notamment un caractère hydrophobe et une résistance aux dégradations biologiques. La granulation est également une application possible après torréfaction de la matière.

La demande FR 3024221 au nom de la demanderesse propose déjà une installation de séchage et de torréfaction de la biomasse avec un rendement énergétique amélioré.

Les produits issus de la torréfaction sont un solide (la biomasse torréfiée) et des gaz de torréfaction dont une fraction, les condensables, est liquide sous des conditions normales de pression et de température.

Dans un procédé de séchage et de torréfaction de la biomasse ligno-cellulosique actuellement connue, on peut distinguer trois étapes principales:
- séchage dans un réacteur de préchauffage/séchage dans lequel la charge d'alimentation de biomasse est chauffée et une partie de l'humidité est éliminée par diffusion et évaporation, comme évoqué ci-dessus ;
- torréfaction dans un réacteur de torréfaction dans lequel la biomasse séchée est chauffée à la température de torréfaction et sous atmosphère en défaut d'oxygène. L'humidité résiduelle est complètement éliminée et un phénomène de dépolymérisation de la biomasse se produit;
- refroidissement avec une cinétique contrôlée dans une unité de refroidissement du produit solide torréfié sous atmosphère inerte. Le produit solide torréfié peut être exposé à l'air en toute sécurité seulement en dessous de 150° C.

Il est connu que parmi les condensables obtenus, certains peuvent être récupérés par un procédé de condensation en vue d'être valorisés. Un procédé de condensation consiste à réaliser le refroidissement d'un flux gazeux multi-composés. Ce refroidissement, qui se fait typiquement dans des échangeurs de chaleur (ou condenseur), permet alors de liquéfier ou de solidifier tout ou partie des différents composés du flux gazeux initial. Selon les gammes opératoires de température du condenseur, il est possible de capter sélectivement les différents composés du flux gazeux et de réaliser ainsi des séparations.

Ainsi, il a été testé différentes matières de biomasse ligno-cellulosique (épicéa, bambou ou bouleau) en vue de récupérer différents types de condensat à valoriser à partir d'une torréfaction : [4]. Ces différents types de condensats sont obtenus à des plages de températures différentes de condensation. Ces condensats sont proposés pour une utilisation directe, en tant que tels, sans privilégier un composé en particulier.

Une approche similaire a été proposée en ciblant cinq produits d'intérêts que sont l'acide acétique, le furfural, l'eugénol, le glycolaldéhyde et le formaldéhyde (formol) :[5]. Les condensats obtenus sont alors séparés et purifiés sans privilégier un produit en particulier, nécessitant ainsi plusieurs opérations d'extraction et de distillation successives afin de pouvoir isoler chacun des produits visés. Cette stratégie propose de mettre en place une condensation étagée où le premier étage de refroidissement à haute température (160°C) permet de récupérer les sucres gênants technologiquement dans la suite des opérations. Un second étage à plus basse température (20°C) délivre un effluent liquide qui sera séparé pour récupérer les autres composés d'intérêt. Il a été alternativement suggéré d'avoir un premier étage à 105°C et -15°C pour le deuxième.

Les procédés de l'état de l'art précités prévoient d'utiliser les gaz libérés lors de la torréfaction pour isoler des condensats et par la suite, récupérer plusieurs molécules d'intérêt ou utiliser directement les condensables en mélange pour divers usages (engrais, insecticide, désherbant). Le document US 2010/223839 A1 décrit aussi un procédé selon l'art antérieur.

Ainsi, aucun procédé ne propose une solution pour récupérer de manière sélective l'eugénol et le cas échéant de formol.

Ces solutions selon l'état de l'art n'envisagent pas non plus la valorisation énergétique des vapeurs sèches non-condensées.

Il existe donc un besoin pour améliorer les procédés de séchage et de torréfaction de biomasse existants, notamment afin de permettre une production sélective d'eugénol et le cas échéant de formol.

Le but de l'invention est de répondre au moins en partie à ce besoin.

### Exposé de l'invention

Pour ce faire, l'invention a pour objet un procédé de production d'eugénol selon la revendication 1.

La biomasse peut être avantageusement de la biomasse ligno-cellulosique.

Grâce à l'invention, on améliore la rentabilité de l'installation de séchage et de torréfaction de la biomasse par rapport aux installations selon l'état de l'art, car des produits à valeur ajoutée sont isolés sélectivement.

En outre, comparativement aux procédés existants qui se sont attachés à une production simultanée de plusieurs produits (le glycolaldéhyde, l'acide acétique, le furfural, l'eugénol), le fait, conformément à la présente invention, de définir une condensation étagée à température de fonctionnement judicieusement sélectionnée pour une production sélective d'eugénol permet de simplifier les trains de distillation ou d'extraction.

Cela a pour avantage d'améliorer les performances énergétiques et baisser le coût d'investissement d'une installation selon l'invention en comparaison d'une installation selon l'état de l'art visant une production simultanée de plusieurs produits.

Dans le réacteur de séchage, la température peut être comprise entre 100 et 300°C, et plus particulièrement entre 100 et 200°C, préférentiellement entre 100 et 110°C. En effet, au-dessus de 200°C les réactions qui ont lieu ne sont plus uniquement des réactions de séchage, et le risque augmente de voir la concentration de goudrons augmenter dans les effluents produits dans le réacteur.

Dans le réacteur de torréfaction, la température de fonctionnement est comprise entre 200 et 310°C. La température est de préférence comprise entre 250 et 310°C. Le choix de cette température peut être ajusté selon la biomasse utilisée mais la température préférentielle pour maximiser la quantité de gaz condensables sans les dégrader thermiquement est avantageusement entre 280°C et 300°C.

A la température de l'étage amont (premier étage), comprise entre 170°C et 230°C, les sucres se condensent et peuvent être séparés facilement.

A la température de récupération de l'eugénol (deuxième étage), comprise entre 110°C et 130°C, et préférentiellement aux environs de 120°C, les sucres ayant été préalablement condensés dans le premier étage, l'eugénol est présent au sein d'une phase aqueuse contenant également d'autres composés organiques (nommés également « autres condensats »), par exemple : Glycolaldéhyde, Acide Acétique ou Furfural. La biomasse peut être tout type de biomasse lignocellulosique. Avantageusement, la biomasse lignocellulosique peut être choisie parmi la paille de blé, le miscanthus et le bois résineux.

Selon une variante avantageuse, le séchage de la biomasse est réalisé à une température comprise entre 100°C et 110°C.

Selon une autre variante avantageuse, la torréfaction de la biomasse séchée est réalisée à une température comprise entre 290°C et 310°C.

De préférence, la biomasse torréfiée est refroidie et humidifiée, de préférence encore par aspersion d'eau.

L 'étape d/ de purification de l'eugénol est une extraction liquide-liquide ou une distillation.

Selon un mode de réalisation avantageux, le procédé comprend en outre une étape de condensation supplémentaire, dans un troisième étage en aval du deuxième étage, la récupération du formaldéhyde étant réalisée en sortie du troisième étage.

L'invention concerne également une installation, destinée à mettre en œuvre un procédé de séchage et de torréfaction décrit précédemment, selon la revendication 8.

Selon un mode de réalisation avantageux, l'installation comprend un étage de condensation supplémentaire alimenté par les vapeurs sortant du deuxième étage de l'unité de condensation étagée, cet étage de condensation supplémentaire étant adaptée pour condenser le formaldéhyde et obtenir du formol, c'est-à-dire une solution aqueuse de formaldéhyde polymérisé avec une teneur globale en formaldéhyde de 37% massique, stabilisée par du méthanol.

L'invention a également pour objet l'utilisation d'une installation décrite ci-avant, pour la production de l'Eugénol et le cas échéant de formaldéhyde.

L'installation selon l'invention peut être intégrée à une installation de conversion thermochimique de biomasse en un gaz de synthèse largement connu sous l'appellation Syngaz, par gazéification dans un réacteur de gazéification en vue de produire un combustible ou un carburant, notamment un carburant liquide, ou un autre produit de synthèse. La synthèse peut être faite selon le procédé Fischer-Tropsch pour produire du gazole liquide ou un autre carburant.

L'installation selon l'invention peut aussi être intégrée à une installation de mise sous forme de granulés de la biomasse torréfiée.

L'installation selon l'invention peut aussi être intégrée à une installation de co-combustion.

### Description détaillée

D'autres avantages et caractéristiques de l'invention ressortiront mieux à la lecture de la description détaillée de l'invention faite à titre illustratif et non limitatif en référence aux figures suivantes parmi lesquelles :
- la figure 1 est une vue d'ensemble schématique d'un exemple d'une installation de séchage, de torréfaction et de condensation selon l'invention pour la production d'eugénol;
- la figure 2 est une vue schématique de la partie aval de l'installation selon la figure 1 ;
- la figure 3 est un graphe illustrant le résultat d'une étude paramétrique économique selon l'état de l'art, du coût de production de la biomasse torréfiée rapporté au prix de vente des produits chimiques issus du traitement de la biomasse torréfiée.

Dans l'ensemble des figures suivantes, la nomenclature suivante est utilisée:
- « Bxx » désigne la biomasse brute ou sèche au cours de son transfert, avec l'indice xx qui représente la quantité en eau dans la biomasse. Par exemple, B35 désigne une biomasse brute avec 35% d'humidité, et B10 une biomasse sèche avec 10% d'humidité. Il va de soi que les valeurs d'humidité sont présentées à titre d'exemple et ne sont pas limitatives. La biomasse concernée est une matière d'origine végétale qui contient du carbone, de l'hydrogène, de l'oxygène et de l'azote et des cendres.
- « Txx » désigne de la même manière le produit solide issu du réacteur de torréfaction avec l'indice xx qui représente la quantité en eau dans la biomasse torréfiée.

Dans la description qui va suivre ainsi que dans l'ensemble de la demande, les termes « entrée », « sortie » « amont », « aval », sont utilisés par référence avec les sens de transfert de la biomasse, de circulation des vapeurs et condensables dans une installation de séchage et de torréfaction de biomasse selon la présente invention.

On décrit ci-après un exemple d'installation 1 en continu mettant en œuvre un séchage suivi d'une torréfaction de biomasse ligno-cellulosique selon l'invention pour produire de l'eugénol. On précise que seule une partie des installations est représentée et qu'elles peuvent comporter bien d'autres unités.

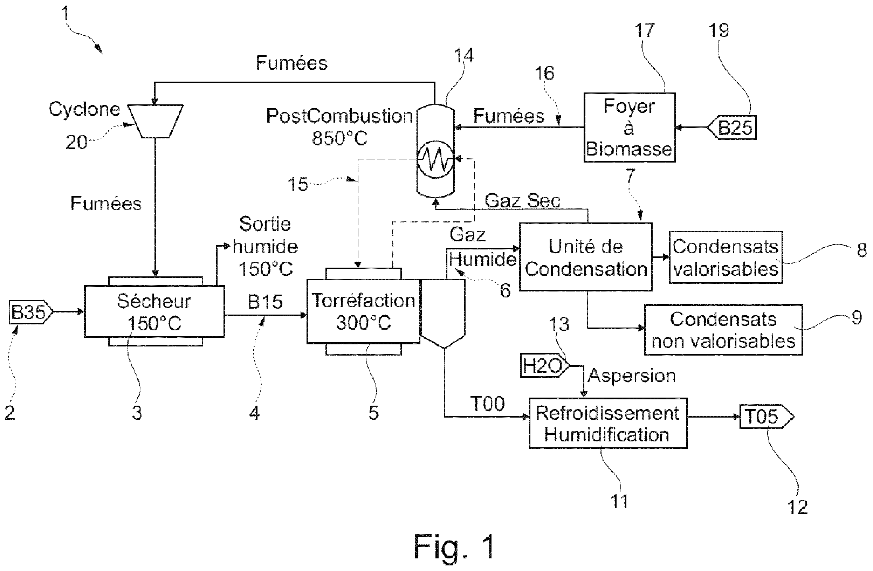

En référence à la figure 1, l'installation 1 selon l'invention est alimentée en entrée par une biomasse humide 2, de préférence B35. Une biomasse avec un taux d'humidité différent peut très bien être envisagée.

La biomasse humide 2 alimente un sécheur 3 adapté pour sécher la biomasse humide à une température d'environ 105°C. Selon un mode de réalisation, le sécheur 3 peut être chauffé par des fumées provenant d'un cyclone 20 et issues de l'installation en aval, comme explicité par la suite.

A la sortie du sécheur 3, la biomasse peut avoir par exemple un taux d'humidité de 15%.

La biomasse séchée 4 est ensuite acheminée dans un réacteur de torréfaction 5.

Le réacteur de torréfaction 5 est adapté pour fonctionner à une température permettant de maximiser la quantité de gaz condensables, sans les dégrader thermiquement. Typiquement, la température de fonctionnement du réacteur de torréfaction 5 est comprise entre 290°C et 310°C, typiquement encore entre 280°C et 300°C.

A la sortie du réacteur de torréfaction 5, les gaz humides 6 sortant sont dirigés vers une unité de condensation étagée 7 permettant de condenser une partie des composés du flux gazeux et de fractionner les condensats obtenus en ceux valorisables 8 et ceux non-valorisables 9.

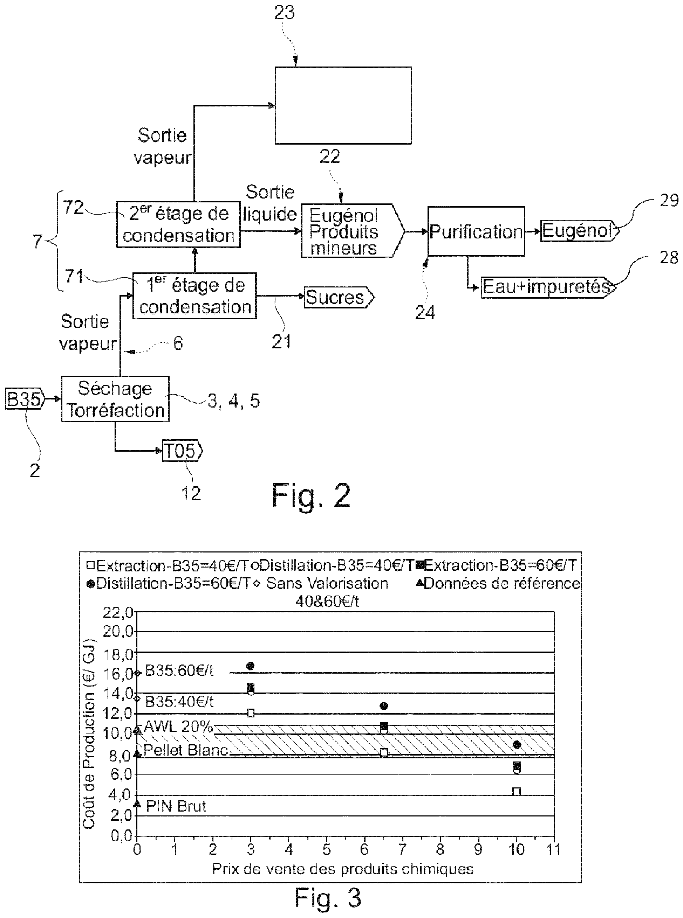

L'unité de condensation 7 adaptée est décrite en détail sur la figure 2 avec l'unité de purification 10 pour obtenir l'eugénol de manière optimale.

La biomasse torréfiée T00 peut quant à elle avoir un taux d'humidité proche 0% en sortie de réacteur de torréfaction 5. Cette biomasse torréfiée peut être acheminée vers une unité de refroidissement et d'humidification 11. L'unité de refroidissement et d'humidification 11 opère préférentiellement sous atmosphère inerte. Elle permet d'obtenir une biomasse torréfiée T05 en sortie de l'installation 12. L'humidification se fait avantageusement par aspersion d'eau 13.

De manière avantageuse, le réacteur de torréfaction 5 peut être chauffé indirectement par un brûleur 14. Ainsi, un élément de récupération de chaleur 15, adapté pour récupérer au moins une partie de la chaleur issue de la combustion qui a lieu dans le brûleur, transfert celle-ci au réacteur de torréfaction 5.

Ce brûleur 14 est préférentiellement alimenté par des fumées 16 provenant d'un foyer à biomasse 17. Le foyer à biomasse peut être alimenté par une biomasse 19 de préférence B25, mais qui peut avoir un % d'humidité différent. Les particules présentes dans les fumées sortant du brûleur 14 sont séparées de celles-ci dans le cyclone 20 qui alimente le sécheur 3.

De manière avantageuse, comme illustré en figure 1, les gaz secs sortant de l'unité de condensation 7 peuvent alimenter directement le brûleur 14.

La figure 2 illustre en détail la partie aval de l'installation 1 selon l'invention. L'unité de condensation étagée 7 comprend deux étages de condensation 71, 72 en série l'un avec l'autre. Selon l'invention, la température de fonctionnement de l'étage 71 est dans une plage entre 170°C et 230°C et la température de fonctionnement de l'étage 72 est dans une plage entre 110°C et 130°C, de préférence aux environs de 120°C. A la température de l'étage 71, les sucres se condensent et peuvent être séparés immédiatement.

Ainsi, les vapeurs sortant 6 du réacteur de torréfaction 5 alimentent le premier étage 71 de l'unité de condensation 7. A la température de fonctionnement indiquée, ce premier étage de condensation 71 permet de condenser les sucres qui peuvent être récupérés indépendamment en sortie 21.

Dans le deuxième étage de condensation 72, en aval du premier 71, l'eugénol et d'autres composés organiques sont condensés puis extraits sous leur forme liquide 22.

Les vapeurs restantes en sortie du deuxième étage de condensation sont envoyées vers un étage de traitement 23. Cet étage de traitement 23 peut être un brûleur pour brûler les vapeurs ou de manière préférée un troisième étage de condensation.

Ce troisième étage de condensation peut permettre de condenser le formaldéhyde. L'étape de purification permet d'obtenir d'un côté l'eugénol 29 et d'un autre côté l'eau et les impuretés 28.

Afin de récupérer exclusivement l'eugénol, une unité de purification 24 est reliée en aval de la sortie du deuxième étage de condensation 72. Cette unité de purification 24 permet de séparer et récupérer de manière distincte l'eugénol d'une part et l'eau et les impuretés mélangées avec l'eau d'autre part.

Selon une alternative préférée, la purification mise en œuvre dans l'unité 24 peut être une extraction liquide-liquide.

Selon une autre alternative, la purification mise en œuvre dans l'unité 24 peut être une distillation.

Dans la suite deux scénarios après le séchage et la torréfaction de la biomasse selon l'état de l'art et selon l'invention sont présentés et évalués comparativement.

Sur le tableau 1 ci-dessous, sont présentés les bilans matières pour différents type de biomasses (Pin (PIN), Frêne (FRENE), Miscanthus (MISC) et Paille de blé (PBLE)) en considérant une production de 5t/h de biomasse torréfiée (T05).

Ces résultats ont été obtenus dans l'objectif de récupérer plusieurs produits à la fois, à savoir le glycolaldéhyde, l'acide acétique, le furfural, l'eugénol et d'autres composés organiques valorisables.

**TABLEAU 1**

| | | | PIN | FRENE | MISC | | PBLE |
|---|---|---|---|---|---|---|---|
| AWL (rendement massique en gaz) | | | 45% | 52% | 52% | | 51% |
| B35 | | | 13,3 | 15,3 | 15,2 | | 15,0 |
| B15 | | | 10,2 | 11,7 | 11,6 | | 11,5 |
| B00 | | | 8,6 | 10,0 | 9,9 | | 9,7 |
| T00 | | | 4,8 | 4,8 | 4,8 | | 4,8 |
| T05 | | | 5,0 | 5,0 | 5,0 | | 5,0 |
| CO+CO2 | | | 0,6 | 1,3 | 1,6 | | 1,8 |
| Condensables | | | 4,8 | 5,7 | 5,3 | | 4,9 |
| | | dont H₂O | 2,9 | 3,4 | 3,4 | | 3,5 |
| | | dont organiques non valorisables | 1,1 | 1,0 | 0,9 | | 0,7 |
| | | | DISTILLATION | | | | |
| *dont organiques valorisables* | | | 0,5 | 0,7 | 0,6 | 0,4 | |
| | *dont Glycolaldéhyde (kg*/*h)* | | 238 | 153 | 124 | 34 | |
| | *dont Acide Acétique (kg*/*h)* | | 186 | 469 | 351 | 259 | |
| | *dont Furfural (kg*/*h)* | | 106 | 104 | 120 | 77 | |
| | *dont Eugénol (kg*/*h)* | | 20 | 14 | 6 | 2 | |
| | | | EXTRACTION | | | | |
| *dont organiques valorisables* | | | 0,5 | 0,7 | 0,6 | 0,4 | |
| | *dont Glycolaldéhyde (kg*/*h)* | | 170 | 109 | 88 | 24 | |
| | *dont Acide Acétique (kg*/*h)* | | 182 | 459 | 343 | 254 | |
| | *dont Furfural (kg*/*h)* | | 111 | 109 | 126 | 81 | |
| | *dont Eugénol (kg*/*h)* | | 29 | 19 | 8 | 3 | |

De ce tableau 1, il ressort que pour 5 t/h de biomasse torréfié T05, correspondant approximativement à 10t/h de biomasse B00, selon les conditions et la biomasse, une production par distillation permet de récupérer de 2 à 20kg d'eugénol et qu'une production par extraction liquide-liquide permet de récupérer de 3 à 29 kg d'eugénol. On peut donc s'attendre approximativement à une fourchette de production de 5 à 25 kg d'eugénol pour 10 tonnes de biomasse B00.

Le tableau 2 montre les résultats de rendement à partir des bilans matière effectués sur le Pin.

**TABLEAU 2**

| Rendements | Avec valorisation Des condensables par EXTRACTION | Sans valorisation Des condensables |
|---|---|---|
| Rendement matière - rapport (T00+Cond)/(B00+B00comb) | 61% | 55% |
| Rendement Matière Condensables Récupérés / B00 | 6% | 0% |
| Rendement Energie - rapport (T00+Cond)/(B00+B00comb+Elec) Extraction | 61% | 64% |

Le meilleur rendement énergétique sans valorisation s'explique par le fait qu'on brûle les condensables non valorisés.

La figure 3 montre sous forme de graphe les résultats d'une étude paramétrique du coût de vente de la biomasse torréfiée en fonction du prix de vente de l'eugénol.

Cette figure 3 montre qu'au-dessus de 6M€/an de revenu lié à l'eugénol (avec un prix du marché entre 8 et 10€/kg), il devient rentable économiquement de valoriser l'eugénol (avec les hypothèses économiques prises par ailleurs).

En effet, le coût de production de la biomasse torréfiée devient alors inférieur aux références de coût de production du pellet de biomasse non-torréfié ou de la biomasse torréfiée sans valorisation des condensables.

L'invention, qui vise à récupérer avant tout l'eugénol, permet de simplifier les trains de distillation ou d'extraction comparativement aux procédés selon l'état de l'art, ce qui présente un avantage sur le plan du bilan énergétique et sur le plan de l'investissement (meilleures performances énergétiques et économiques comparées aux procédés selon l'état de l'art qui visent une production concomitante de plusieurs produits).

En outre, l'étude paramétrique prouve aussi l'intérêt économique d'un point de vue coût de production.

D'autres variantes ou améliorations peuvent être prévues sans pour autant sortir du cadre de l'invention.

### Références citées

[1]: Haarlemmer G, Boissonnet G, Imbach J, Setier P-A, Peduzzi E. « Second génération BtL type biofuels - a production cost analysis ». Energy & Environmental Science 2012; 7.
[2]: Simpson WT. « Equilibrium Moisture Content of Wood in Outdoor Locations in the United States and Worldwide ». FPL-RN-0268. Forest Products Laboratory: Madison, Wisconsin, 1998.
[3]: Höldrich A, Hartmann H. « In Storage of wood logs - «Drying speed and dry matter losses », 14th European Biomass Conference, Paris, France, 2005.
[4]: Fagernäs, Leena, Eeva Kuoppala, Vesa Arpiainen. « Composition, utilization and economic assessment of torrefaction condensates. » Energy & Fuels, 2015, 29.5 :3134-3142.
[5]: Detcheberry, Mylene. « Valorisation chimique des condensats issus de la torréfaction de biomasses: modélisation thermodynamique, conception et analyse des procédés. » Diss. Toulouse, INPT, 2015.

## Revendications

1. Procédé de production d'eugénol comportant les étapes suivantes :
a/ séchage d'une biomasse humide;
b/ torréfaction de la biomasse séchée selon l'étape a/ dans un réacteur de torréfaction à une température de fonctionnement comprise entre 200°C et 310°C;
c/ condensation étagée, des vapeurs issues de la torréfaction selon l'étape b/, la condensation étant réalisée dans au moins deux étages en série à des températures comprises entre 170 et 230°C dans le premier étage et entre 110 et 130°C dans le deuxième étage, en aval du premier étage, la récupération des sucres liquides étant réalisée en sortie du premier étage, tandis que la récupération du mélange liquide constitué de l'eugénol et des autres condensats étant réalisée en sortie du deuxième;
d/ purification du mélange récupéré selon l'étape c/, afin de récupérer l'eugénol, l'étape d/ de purification de l'eugénol étant une extraction liquide-liquide ou une distillation.

2. Procédé selon la revendication 1, la biomasse étant de la lignocellulose.

3. Procédé selon la revendication 2, la biomasse lignocellulosique étant choisie parmi la paille de blé, le miscanthus et le bois résineux.

4. Procédé selon l'une quelconque des revendications 1 à 3, selon lequel le séchage de la biomasse est réalisé à une température comprise entre 100°C et 110°C.

5. Procédé selon l'une quelconque des revendications précédentes, selon lequel la torréfaction de la biomasse séchée est réalisée à une température comprise entre 290°C et 310°C.

6. Procédé selon l'une quelconque des revendications précédentes, selon lequel la biomasse torréfiée est refroidie et humidifiée, de préférence par aspersion d'eau.

7. Procédé selon l'une quelconque des revendications précédentes, comprenant en outre une étape de condensation supplémentaire, dans un troisième étage en aval du deuxième étage, la récupération du formaldéhyde étant réalisée en sortie du troisième étage.

8. Installation, destinée à mettre en œuvre un procédé de séchage et de torréfaction selon l'une quelconque des revendications 1 à 7, comprenant :
- un réacteur de séchage, adapté pour sécher de la biomasse humide,
- un réacteur de torréfaction relié au réacteur de séchage et adapté pour réaliser la torréfaction de la biomasse séchée en amont, à une température de fonctionnement comprise entre 200°C et 310°C,
- une unité de condensation comprenant deux étages de condensation en série le premier étage étant alimenté par les vapeurs sortant du réacteur de torréfaction, les deux étages en série étant adaptés pour fonctionner à une température comprise entre 170°C et 230°C pour le premier étage et entre 110 et 130°C pour le deuxième étage en aval du premier étage;
- une unité de purification des condensats sortant du deuxième étage comprenant une colonne de distillation ou une colonne d'extraction liquide-liquide.

9. Installation selon la revendication 8, comprenant un étage de condensation supplémentaire alimenté par les vapeurs sortant du deuxième étage de l'unité de condensation étagée, cet étage de condensation supplémentaire étant adaptée pour condenser le Formol.

10. Utilisation d'une installation selon l'une quelconque des revendications 8 à 9, pour la production de l'Eugénol et le cas échéant de formaldéhyde.

## Patentansprüche

1. Verfahren zur Herstellung von Eugenol mit den folgenden Schritten:
a/ Trocknen einer feuchten Biomasse;
b/ Torrefikation der gemäß Schritt a/ getrockneten Biomasse in einem Torrefikationsreaktor bei einer Betriebstemperatur zwischen 200 °C und 310 °C;
c/ stufenweise Kondensation der aus der Torrefikation gemäß Schritt b/ herrührenden Dämpfe, wobei die Kondensation in mindestens zwei in Reihe angeordneten Stufen bei Temperaturen zwischen 170 und 230 °C in der ersten Stufe und zwischen 110 und 130 °C in der zweiten Stufe, stromabwärts der ersten Stufe, durchgeführt wird, wobei die Rückgewinnung von flüssigen Zuckern am Ausgang der ersten Stufe erfolgt, während die Rückgewinnung des aus Eugenol und anderen Kondensaten bestehenden flüssigen Gemischs am Ausgang der zweiten erfolgt;
d/ Reinigung des gemäß Schritt c/ gewonnenen Gemischs, um Eugenol zu gewinnen, wobei Schritt d/ der Reinigung von Eugenol eine Flüssig-Flüssig-Extraktion oder eine Destillation ist.

2. Verfahren nach Anspruch 1, wobei es sich bei der Biomasse um Lignocellulose handelt.

3. Verfahren nach Anspruch 2, wobei die Lignocellulose-Biomasse aus Weizenstroh, Miscanthus und Weichholz ausgewählt ist.

4. Verfahren nach einem der Ansprüche 1 bis 3, wobei das Trocknen der Biomasse bei einer Temperatur zwischen 100 °C und 110 °C durchgeführt wird.

5. Verfahren nach einem der vorhergehenden Ansprüche, wobei die Torrefikation der getrockneten Biomasse bei einer Temperatur zwischen 290 °C und 310 °C durchgeführt wird.

6. Verfahren nach einem der vorhergehenden Ansprüche, wobei die torrefizierte Biomasse abgekühlt und, vorzugsweise durch Besprühen mit Wasser, angefeuchtet wird.

7. Verfahren nach einem der vorhergehenden Ansprüche, das außerdem einen zusätzlichen Kondensationsschritt in einer dritten Stufe, stromabwärts der zweiten Stufe, umfasst, wobei die Rückgewinnung von Formaldehyd am Auslass der dritten Stufe erfolgt.

8. Anlage zur Durchführung eines Trocknungs- und Torrefikationsverfahrens nach einem der Ansprüche 1 bis 7, umfassend:
- einen Trocknungsreaktor, der zum Trocknen von feuchter Biomasse geeignet ist,
- einen Torrefikationsreaktor, der mit dem Trocknungsreaktor verbunden und dazu geeignet ist, die Torrefikation der stromaufwärts getrockneten Biomasse bei einer Betriebstemperatur zwischen 200 °C und 310 °C durchzuführen,
- eine Kondensationseinheit, die zwei in Reihe angeordnete Kondensationsstufen umfasst, wobei die erste Stufe mit den aus dem Torrefikationsreaktor herausgeführten Dämpfen beschickt wird, wobei die beiden in Reihe angeordneten Stufen für einen Betrieb bei einer Temperatur zwischen 170 °C und 230 °C für die erste Stufe und zwischen 110 und 130°C für die zweite Stufe, stromabwärts der ersten Stufe, ausgelegt sind;
- eine Einheit zum Reinigen der aus der zweiten Stufe herausgeführten Kondensate, die eine Destillationskolonne oder eine Flüssig-Flüssig-Extraktionskolonne umfasst.

9. Anlage nach Anspruch 8, umfassend eine zusätzliche Kondensationsstufe, die mit den aus der zweiten Stufe der Stufenkondensationseinheit herausgeführten Dämpfen beschickt wird, wobei diese zusätzliche Kondensationsstufe zum Kondensieren von Formalin ausgelegt ist.

10. Verwendung einer Anlage nach einem der Ansprüche 8 bis 9 zur Herstellung von Eugenol und gegebenenfalls Formaldehyd.

## Claims

1. Process for the production of eugenol comprising the following steps:
a/ drying of a wet biomass;
b/ torrefaction of the biomass dried according to step a/ in a torrefaction reactor at an operating temperature of between 200°C and 310°C;
c/ staged condensation of the vapours resulting from the torrefaction according to step b/, the condensation being carried out in at least two stages in series at temperatures of between 170 and 230°C in the first stage and between 110 and 130°C in the second stage, downstream of the first stage, the recovery of the liquid sugars being carried out at the outlet of the first stage, while the recovery of the liquid mixture consisting of the eugenol and of the other condensates is carried out at the outlet of the second;
d/ purification of the mixture recovered according to step c/, in order to recover the eugenol, step d/ of purification of the eugenol being a liquid/liquid extraction or a distillation.

2. Process according to Claim 1, the biomass being lignocellulose.

3. Process according to Claim 2, the lignocellulosic biomass being chosen from wheat straw, miscanthus and coniferous wood.

4. Process according to any one of Claims 1 to 3, according to which the drying of the biomass is carried out at a temperature of between 100°C and 110°C.

5. Process according to any one of the preceding claims, according to which the torrefaction of the dried biomass is carried out at a temperature of between 290°C and 310°C.

6. Process according to any one of the preceding claims, according to which the torrefied biomass is cooled and moistened, preferably by spraying with water.

7. Process according to any one of the preceding claims, also comprising an additional condensation step, in a third stage downstream of the second stage, the recovery of formaldehyde being carried out at the outlet of the third stage.

8. Plant, intended to carry out a drying and torrefaction process according to any one of Claims 1 to 7, comprising:
- a drying reactor, suitable for drying wet biomass,
- a torrefaction reactor connected to the drying reactor and suitable for carrying out the torrefaction of the biomass dried upstream, at an operating temperature of between 200°C and 310°C,
- a condensation unit comprising two condensation stages in series, the first stage being fed by the vapours exiting from the torrefaction reactor, the two stages in series being suitable for operating at a temperature of between 170°C and 230°C for the first stage and between 110 and 130°C for the second stage downstream of the first stage,
- a unit for purification of the condensates exiting from the second stage comprising a distillation column or a liquid/liquid extraction column.

9. Plant according to Claim 8, comprising an additional condensation stage fed by the vapours exiting from the second stage of the staged condensation unit, this additional condensation stage being suitable for condensing formalin.

10. Use of a plant according to either one of Claims 8 and 9 for the production of eugenol and, if appropriate, of formaldehyde.
